## Europäisches Patentamt
## European Patent Office
## Office européen des brevets

(19)

(11) Publication number: **0 047 009**
**A1**

(12)

# EUROPEAN PATENT APPLICATION

(21) Application number: **81106798.2**

(22) Date of filing: **01.09.81**

(51) Int. Cl.³: **C 08 F 220/06**
**A 61 L 15/00, C 08 F 2/46**
**//(C08F220/06, 212/00, 210/14)**

(30) Priority: **02.09.80 US 183616**

(43) Date of publication of application:
**10.03.82 Bulletin 82/10**

(84) Designated Contracting States:
**BE CH DE FR GB IT LI NL SE**

(71) Applicant: **The B.F. GOODRICH Company**
**Dept. 0015 WHB-6 500 South Main Street**
**Akron, Ohio 44318(US)**

(72) Inventor: **George, Paul John**
**3429 Boston Mills Road**
**Richfield Ohio 44286(US)**

(74) Representative: **von Kreisler, Alek et al,**
**Deichmannhaus am Hauptbahnhof**
**D-5000 Köln 1(DE)**

(54) **Hydrophilic interpolymers of acrylic acid and an olefin or a styrene.**

(57) Highly water absorbent polymers are obtained by polymerizing 70 to 93 weight percent of acrylic acid and 7 to 30 weight percent of a comonomer selected from α-olefins having 6 to 18 carbon atoms, styrene and substituted styrenes. The polymerization can be carried out in the presence of a free radical catalyst in which case the polymer is thereafter preferably neutralized with a base. Alternatively, carboxylic groups are neutralized prior to polymerization by subjecting the monomer mixture either to radiation or to UV light in the presence of a photoinitiator. Such polymers are useful in the manufacture of articles which absorb water or body fluids.

EP 0 047 009 A1

0047009

-1-

## HYDROPHILIC INTERPOLYMERS
## OF ACRYLIC ACID AND AN
## OLEFIN OR A STYRENE

### BACKGROUND OF THE INVENTION

A variety of hydrophilic polymers which are useful in the manufacture of water absorbent films and fibers have been reported in the prior art. U.S. Patent 3,915,921 discloses copolymers of unsaturated carboxylic acid monomers with alkyl acrylate esters wherein the alkyl group contains 10 to 30 carbon atoms. However, because of the high Tg of these polymeric materials, it is difficult to extrude them in fiber or film form. Furthermore, films pressed from the powders require high temperatures, the films are brittle and fragile, and have a reduced initial rate of water absorption.

U.S. Patent 4,062,817 discloses polymers of unsaturated copolymerizable carboxylic acids, at least one alkyl acrylate or methacrylate wherein the alkyl group has 10 to 30 carbon atoms and another alkyl acrylate or methacrylate wherein the alkyl group has 1 to 8 carbons. This composition alleviated many of the deficiencies of the earlier compositions. Further improvements in the hydrophilic properties were obtained by compositions disclosed in U.S. Patent 4,066,583. This patent discloses a composition comprising (1) a copolymer of the type disclosed in the '817 patent, except that after copolymerization 30 to 90 percent of the carboxylic groups were neutralized with an alkali metal or ammonia and (2) an aliphatic glycol, a plasticizer which is important in facilitating extrusion of the polymer.

Most recently, U.S. Patent 4,167,464 discloses highly water absorbent polymers obtained by photopoly-
merizing an alkaline metal salt of acrylic acid, a
long chain alkyl acrylate or methacrylate and a short
chain alkyl acrylate or methacrylate in the presence of
a photoinitiator.

Such prior art water absorbent films and
fibers have applications in disposable, non-woven uses,
such as in disposable diapers, medical-surgical supplies
and personal care products. Often it would be useful
to incorporate such highly absorbent polymers into
woven, washable diapers or garments. However, commercial washing methods subject the washable items to
high temperatures (up to 77°C during washing and 120°C
during drying) and both basic (pH 10) and acidic
(pH 5.5) conditions. Under such conditions the prior
art polymers would undergo hydrolysis which would result
in the loss of absorbency properties.

SUMMARY OF THE INVENTION

A highly water absorbent interpolymer which
is resistant to hydrolysis obtained from a monomer
mixture of 65 to 90 weight percent of acrylic acid,
70 to 100% of the carboxylic groups of said acid having
been neutralized prior to polymerization with an
alkaline metal hydroxide or ammonia and 10 to 35
weight percent of an α-olefin, having 6 to 18 carbon
atoms styrene or a substituted styrene. The monomer
mixture can be either spread to the desired thickness
or spun into a fiber and then polymerized upon exposure
to a UV light or radiation sources. If photopolymerized,
a photoinitiator must be employed. Alternatively, the
monomer mixture, where the acrylic acid had not been
previously neutralized, can be polymerized in the
presence of a free radical peroxygen catalyst.

-3-

## DETAILED DISCLOSURE

This invention is directed to an interpolymer which has outstanding absorption and retention properties of water and ionic solutions such as urine or blood. The interpolymer is prepared from a monomer mixture comprising

a) 70 to 93 weight percent of acrylic acid, 70 to 100 percent, and most preferably 80 to 100 percent, of the carboxylic groups having been neutralized with an alkali metal hydroxide or ammonia base prior to polymerization, and

b) 7 to 30 weight percent of a comonomer selected from the group consisting of an α-olefin of 6 to 18 carbon atoms, styrene or a substituted styrene. Substituted styrenes are selected from vinyl toluene, p-halostyrene such as p-bromostyrene, p-iodostyrene, p-fluorostyrene and especially p-chlorostyrene, p-lower alkyl styrene such as p-methylstyrene, p-ethylstyrene, p-n-propylstyrene, p-isopropylstyrene and especially p-t-butylstyrene. Illustrative examples of α-olefins are hexene, heptene, octene, decene, dodecene, tetradecene, hexadecene, octadecene and the like. A mixture of α-olefins may also be employed. The useful mixture may contain an appreciable amount of isomers which have internal unsaturations. Although such mixtures are not preferred, they yield novel interpolymers having very useful properties.

The monomer mixture may be polymerized by subjecting it to radiation such as gamma rays or electron beam or it may be photopolymerized in which case 0.01 to 5 weight percent of a photoinitiator is also employed.

It should be noted that especially the α-olefin

comonomers do not generally copolymerize fully with the acrylic acid monomer. Therefore, usually the resulting copolymer has a somewhat lower α-olefin content than that contained in the monomer mixture. The exact percentage of the α-olefin which actually copolymerizes will depend on the specific α-olefin employed, the polymerization method used, the catalyst or the photoinitiator employed and/or the amount and source of radiation or UV light to which the monomers are subjected, as well as the polymerization conditions. It may be generally stated that from 40 to 60 percent of the α-olefin comonomer is copolymerized. Therefore, preferred interpolymers comprise from 80 to 95 weight percent of acrylic acid monomer units and 5 to 20 weight percent of an α-olefin or styrene comonomer units. Most carboxylic groups in the acrylic acid monomer units are neutralized, but since the degree of neutralization may be less than 100 percent, there may be some free carboxylic groups present in the acrylic monomer units.

In addition to the above discussed monomers from which the copolymers of this invention are prepared, minor amounts, that is less than 5 weight percent, of additional comonomers may also be used. Whether these additional monomers are employed will depend on the end use and the physical properties required, that is, the speed and degree of absorption and the tear strength needed for the film or fabric. Such additional comonomers are various methacrylates, acrylates or nitriles.

Useful alkyl methacrylates are those where the alkyl group has 1 to 30 carbon atoms and preferably 1 to 4 carbon atoms. Representative examples of such methacrylates are methyl methacrylate, ethyl methacrylate, n-butyl methacrylate, n-hexyl methacrylate, n-octyl methacrylate, n-decyl methacrylate, i-decyl methacrylate, lauryl methacrylate, stearyl methacrylate, eicosyl methacrylate, octaeicosyl methacrylate and the like. Examples of cycloalkyl methacrylates are cyclohexyl methacrylate, which is preferred, 1-methylcyclohexyl methacrylate, 1,3-dimethylcyclohexyl methacrylate, 1,3,5-trimethylcyclohexyl methacrylate, 1-ethylcyclohexyl methacrylate, 1-butylcyclohexyl methacrylate, 1,3,5-triethylcyclohexyl methacrylate, 1,3,5-tributylcyclohexyl methacrylate and the like. Illustrative examples of useful acrylates are phenyl acrylate; phenoxyalkyl acrylates such as phenoxyethyl acrylate, which is preferred, phenoxybutyl acrylate, phenoxypropyl acrylate, phenoxyhexyl acrylate. Examples of dialkylaminoalkyl acrylates and methacrylates are dimethylaminoethyl acrylate, which is preferred, dimethylaminobutyl acrylate, dimethylaminohexyl acrylate, diethylaminoethyl acrylate, diethylaminobutyl acrylate, dipropylaminohexyl acrylate, dipropylaminopropyl acrylate, dibutylaminoethyl acrylate, dibutylaminobutyl acrylate, dibutylaminohexyl acrylate, di-n-hexylaminoethyl acrylate, di-n-octylaminoethyl acrylate, di-n-octylaminobutyl acrylate, dimethylaminoethyl methacrylate, dimethylaminobutyl methacrylate, dimethylaminohexyl methacrylate, diethylaminoethyl methacrylate, diethylaminobutyl methacrylate, di-n-octylaminohexyl methacrylate and the like.

Other useful comonomers are α,β-olefinically unsaturated nitriles, preferably the monoolefinically unsaturated nitriles, having from 3 to 10 carbon atoms such as acrylonitrile, methacrylonitrile, ethacrylonitrile, and the like. Most preferred nitriles are acrylonitriles and methacrylonitriles.

The above discussed monomers can be copoly-

BAD ORIGINAL

-6-

merized by subjecting the monomer mixture to UV light. If a film is desired the monomer can be spread on a surface to the desired thickness, e.g. 1 mil to 25 mil, and then subjected to UV light for a short time, e.g. one second to several minutes. The actual length of irradiation will depend on a number of factors, such as the thickness of the monomer film, the distance from and the intensity of the source of irradiation, the specific monomers employed and the ratio of such monomers to each other, the presence or absence of additional comonomers and the nature and the amount of the photoinitiator employed. The type of photoinitiator employed will depend at least in part on the type of UV irradiation employed (particularly its wave length) since various photoinitiators may be decomposed by UV light of different wavelengths. If it is desired that the material be in the form of fibers, the monomer mixture can be thickened and then spun into fibers which, upon exposure to UV light, are polymerized.

In order to effect quick and efficient polymerization under UV light, 0.01 to 5 weight percent of a photoinitiator, preferably 0.1 to 5 percent and more preferably 0.3 to 1.0 weight percent, must be incorporated into the monomer mixture. Any compound which dissociates into free radicals when exposed to UV radiation can be employed. There are many known photoinitiators or photosensitizers such as acetophenone, propiophenone, benzophenone, xanthone, fluorenone, benzaldehyde, fluorene, anthraquinone, triphenylamine, carbazole, 3- or 4-methylacetophenone, 3- or 4-pentylacetophenone, 3- or 4-methoxyacetophenone, 3- or 4-bromoacetophenone, 3- or 4-allylacetophenone, p-di-acetylbenzene, 3- or 4-methoxybenzophenone, 3- or 4-methylbenzophenone, 3- or 4-chlorobenzophenone, 4,4-dimethoxybenzophenone, 4-chloro-4'-benzylbenzophenone, 3-chloroxanthone, 3,9-dichloroxanthone, 3-chloro-8-nonyl-xanthone, 3-methoxyxanthone, 3-iodo-7-methoxyxanthone, 2,2-dimethoxyacetophenone, 2,2-dimethoxy-2-phenylacetophenone, 2,2-diethoxyacetophenone, 2,2-dibutoxyacetophenone, 2,2-dihexoxyacetophenone, 2,2-di(2-ethylhexoxy) acetophenone, 2,2-diphenoxyacetophenone, benzoin, methyl benzoin ether, ethyl bezoin ether, isopropyl benzoin ether, butyl benzoin ether, isobutyl benzoin ether, benzoin acetate, benzoin phenyl carbamate, α,α-diethoxyacetophenone, α,α-diethoxy-a-phenyl-acetophenone, α,α-dimethoxy-a-phenylacetophenone, 4,4'-dicarboethoxybenzoin ethyl ether, α-chloroacetophenone, α-bromoacetophenone, benzoin phenyl ether, α-methylbenzoin ethyl ether, benzoin acrylate, α-methylolbenzoin methyl ether, α,α,α-trichloroacetophenone, o-bromoacetophenone, 4-(benzoylphenylmethoxycarbonylimino)-2-(acrylyloxyethoxycarbonylimino)-1-methylbenzene, cumene hydroperoxide, benzoyl peroxide, dicumyl peroxide, tert-butyl perbenzoate, α,α-azobisisobutyronitrile, phenyl disulfide, chloromethylbenzanthrone, chloromethylanthraquinine, chloromethylnaphthalene, bromomethylbenzanthrone, bromomethylanthraquinone, bromomethylnaphthalene, and the like, and mixtures thereof.

In addition to the photoinitiator it may be advantageous to employ also from 0.3 to 5.0 percent of an activator. Illustrative examples of such activators are mercaptoacetic acid, mercaptoethanol, or organic amines such as methylamine, decylamine, diisopropylamine, tributylamine, tri-2-chloroethylamine, ethanolamine, triethanolamine, methyldiethanolamine, 2-aminoethyl-ethanolamine, allylamine, cyclohexylamine, cyclopenta-dienylamine, diphenylamine, ditolylamine, trixylylamine, tribenzylamine, N-cyclohexylethyleneimine, piperidine, 2-methylpiperidine, N-ethylpiperidine, 1,2,3,4-tetrahydropyridine, 2- or 3- or 4-picoline, morpholine, N-methyl-

BAD ORIGINAL

morpholine, piperazine, N-methylpiperazine, 2,2-dimethyl-1,3-bis-[3-(N-morpholinyl)propionyloxy]-propane, 1,5-bis [3-(N-morpholinyl)propionyloxy]di-ethyl ether, N,N-dimethylaniline and the like.

The monomer mixtures are prepared as aqueous dispersions which eliminates the need for organic solvents. This avoids the pollution problems caused by the removal of organic solvents or the cost associated with the removal of the pollutants. In order to obtain a stable homogeneous dispersion of the monomers, it is preferred that the aqueous dispersions contain 0.01 to 5%, and preferably 0.1 to 1%, of a surface active agent such as an anionic, amphoteric, or nonionic dispersing agent or a mixture of dispersants. Useful anionic dispersing agents include alkali metal or ammonium salts of the sulfates of alcohols having from 8 to 18 carbon atoms such as sodium lauryl sulfate; ethanolamine lauryl sulfate, ethylamine lauryl sulfate; alkali metal and ammonium salts of sulfonated petroleum and paraffin oils; sodium salts of aromatic sulfonic acids such as dodecane-1-sulfonic acid and octadecane-1-sulfonic acid; aralkyl sulfonates such as sodium isopropyl benzene sulfonate, sodium dodecyl benzene sulfonate and sodium isobutyl naphthalene sulfonate; alkali metal and ammonium salts of sulfonated dicarboxylic acid esters such as sodium dioctyl sulfosuccinate, disodium-n-octadecyl sulfosuccinate; alkali metal or ammonium salts of free acid of complex organic mono-and diphosphate esters, sulfosuccinic acid derivatives (AEROSOL dispersants), organic phosphate esters (GAFAC dispersants) and the like. Nonionic dispersants such as octyl-or nonylphenyl poly-ethoxyethanol as well as the PLURONIC and the TRITON dispersants may also be used. Also useful are amphoteric

-9-

dispersants such as dicarboxylic coconut derivatives (MIRANOL). Further examples· of useful dispersants are those disclosed beginning on page 102 in J. Van Alphen's "Rubber Chemicals", Elsevier Publishing Co., 1956.

The monomer mixture can also be polymerized without first neutralizing the carboxylic groups. The polymerization can be carried out in an inert diluent having some solubilizing action on one or more of the monomeric ingredients. Polymerization in mass may be employed but is not preferred because of the difficulty in working up the solid polymeric masses obtained. Polymerization in an aqueous medium containing a watersoluble free radical peroxide catalyst is useful. Polymerization in an organic liquid which is a solvent for the monomers but a non-solvent for the polymer, or in a mixture of such solvents, in the presence of a solvent-soluble catalyst is more preferred because the product is usually obtained as a very fine friable and often fluffy precipitate which, after solvent removal, seldom requires grinding or other treatment before use. The polymerizations preferably are conducted in the presence of a haloethane or halomethane containing at least four halogen atoms. Representative materials include for example, a fluoroethane, fluoromethane, chlorofluoromethane, bromofluoroethane, or preferably a chlorofluoroethane or chlorofluoromethane containing at least four halogen atoms.

Polymers obtained from free radical polymerizations generally do not attain their maximum properties until converted to a partial alkali, ammonium or amine salt. The neutralizing agent is preferably a monovalent alkali such as sodium, potassium, lithium or ammonium hydroxide or the carbonates and bicarbonates thereof, or mixtures of the same, and also amine bases having not

-10-

more than one primary, or secondary amino group. Such amines include, for example, triethanolamine, ethanolamine, isopropanolamine, triethylamine, trimethylamine, and the like.

The procedures to be employed in free radical polymerizations of such monomers, neutralization of the resulting polymers and their work up is disclosed in greater detail in U.S. Patent 4,062,817, which disclosure is incorporated herein by reference.

Although cross-linking agents are not required to obtain useful, highly absorbent compositions of this invention, it may be desirable to incorporate a cross-linking agent since films prepared from compositions containing a cross-linking agent tend to have greater gel strength and an improved ability for the copolymers to swell under a confining pressure. Cross-linking agents may be used in the concentration of about 0.01 to about 20% by weight based on the total weight of the monomers, and preferably about 0.1 to about 5%.

Useful cross-linking monomers are polyalkenyl polyethers having more than one alkenyl ether grouping per molecule or monomers which contain two to six ethylenically unsaturated groups such as allyl, acrylate, or vinyl groups. The most useful possess alkenyl groups in which an olefinic double bond is attached to a terminal methylene group, $CH_2=C<$. Other cross-linking monomers include, for example diallyl esters or ethers, allyl or methallyl acrylates and acrylamides, diacrylates and dimethacrylates, divinyl compounds and the like. Illustrative examples of polyfunctional cross-linking agents are polyethyleneglycol diacrylate and dimethacrylate, ethylene glycol dimethacrylate, tetraethyleneglycol diacrylate, 1,3-butyleneglycol dimethacrylate, diethyleneglycol divinyl ether, trimethy-

-11-

lolpropane diallyl ether, divinyl benzene, trimethylolpropane triacrylate, trimethylolpropane trimethacrylate, triallyl cyanurate, pentaerythritol triacrylate, diallyl itaconate, methylene bis(acrylamide), allyl pentaerithritol, allyl sucrose, 1,6-hexanediol diacrylate, tetramethylene glycol diacrylate and dimethacrylate, ethylene glycol diacrylate and dimethacrylate, triethylene glycol dimethacrylate, triallyl cyanurate, triallyl isocyanurate, diallyl itaconates and the like.

As discussed above, the interpolymers of this invention can be photopolymerized. Additionally, these interpolymers can be obtained by radiation polymerization by subjecting said monomers to electron beam radiation of sufficient intensity to cause said monomers to polymerize substantially completely. The amount and the intensity of radiation required will depend on the thickness of the film, the specific monomers employed, and the speed and the degree of polymerization desired. Generally, for the applications for which the resulting polymers are especially useful films, sheets or fibers in the range from 0.5 to 5 mils are most desirable. Therefore, relatively low intensity electron beam sources, generally less than 200 KV, would be sufficient to effect polymerization. Generally for the type of monomer systems employed in this invention from 1 to 15 M rads of radiation is required. However, it should be pointed out that the amount and intensity of radiation must be optimized for each system taking all variables into consideration, i.e., the monomers employed, the thickness of the film, the desired speed of polymerization, the desired degree of polymerization and the rate of radiation.

When employing photopolymerization or radiation polymerization methods, the polymers of this invention can be polymerized in a film or a fiber form. The

-12-

resulting film or fiber is an elastic, flexible material that has an appreciable degree of strength. If a fine, flaky form is desired, the film can be converted to such a form by drying and then pulverizing or grinding it in standard equipment.

As water absorbent materials these polymers find many uses in film, fiber, fabric and similar forms. They are of particular utility in the disposable non-woven industry where there is need for polymers which will absorb and retain water and ionic physiological fluids. An important feature of these polymers is their enhanced thickening property even in the presence of a salt. Specific applications include disposable diapers, medical-surgical supplies and personal care products. Such applications require a polymer which must imbibe the liquid to be absorbed rapidly and be a polymer that will not dissolve. Further, the fluid must be immobilized or congealed in some way to be retained. The materials may also be used as suitable additives to greatly increase the absorptive power of conventional absorbents such as cotton, wood pulp and other cellulosic absorbents used in applications such as wiping cloths, surgical sponges, catamenial devices, and the like. In a specific application, for example, a disposable diaper, there is an inner layer of a soft absorbent non-woven material that absorbs and passes urine to an inner layer of fluffy fibrous absorbent material, wherein during the construction of this non-woven fiber agglomerates or fibers of the polymers of this invention may be included and an additional impervious plastic layer, as polyethylene. A film of the copolymers of this invention may be used between the outer plastic layer and the inner fluffy absorbent layer. Use of the polymers of this invention can result in reduction in the bulk size of many disposable non-

-13-

wovens.

The instant copolymers can also be used as flocculants in water treatment, in metallurgical processes, in ore beneficiation and flotation, in agricultural applications such as in soil treatment or seed coating or in any applications where the inherent properties of the polymer are desirable, such as its thickening property in an aqueous medium.

To prepare the cured copolymers of this invention, the monomers, a dispersant and a photoinitiator, if used, are mixed in a vessel. Then either a film or fibers are produced from the monomer mixture which, upon exposure to UV light or radiation, are rapidly polymerized. The various steps in the procedure are described in greater detail below.

Monomer Mixture Preparation: The monomer mixture can be prepared by following one of two simple procedures. One method is to dissolve a previously prepared and dried alkali metal or ammonium salt of acrylic acid in water to which is then added a dispersant. To the aqueous solution is then added an $\alpha$-olefin or a styrene monomer which already contains a photoinitiator if one is employed. Another method is to prepare the acrylic acid salt in situ by adding acrylic acid to the proper amount of cold aqueous base (e.g. KOH, NaOH or $NH_4OH$) with cooling. To the aqueous solution is then added a mixture of the $\alpha$-olefin or a styrene monomer to which, if required, a photoinitiator was previously added; the dispersant is added last.

Film Preparation: The aqueous monomer dispersion is spread to a desired thickness (e.g. by the use of Boston-Bradley adjustable blade, by spraying or other known means) on a suitable substrate (e.g. Mylar, polyethylene, paper, etc.). The liquid film is then exposed to a UV or radiation source which polymerizes the monomer mixture into a soft, pliable form.  If desired, this film can be dried in an oven at about 50°C for 1 to 15 min.  After drying the film may still retain some flexibility or become brittle and flaky, depending on the length of drying.

Fiber Preparation:  The aqueous monomer dispersion is thickened to the desired degree with a non-reactive thickening agent such as a cellulose derivative as, for example, hydroxypropyl cellulose, high molecular weight polyvinyl pyrrolidone and the like; natural gums such as guar gum, locust bean gum, gum tragacanth; agar, naturally occuring hydrocolloids such as alginates and the like.  Fibers are then spun from a spinneret in a regular manner and immediately exposed to a UV or radiation source.

To further illustrate the present invention the following examples are presented.  The copolymers and the films were prepared according to the procedures described above.  The copolymers of Examples 1 to 9, presented in Table I, have been photopolymerized using QC 1202 Processor manufactured by Radiation Polymer Co. (with belt speed of 0 to 1000 ft/min-304.8 m/min) having 2 medium pressure quartz mercury vapor lamps at 200 watts/lineal inch (watts/lineal 2.54 cm.).  The distance from the lamps to the film was 15 cm. and the exposure time was 20 sec. at belt speed of 20 ft/min. (6 m/min.).

Comonomers employed in the Examples and identified in Table I by capital letters A to F, are identified below:

A - Styrene

B - Vinyl toluene

C - p-Chlorostyrene

D - p-t-Butylstyrene

0047009

-15-

E - 1 and 2-Octene (mixture of isomers-
   Eastman product P 1821)
F - 1-Octadecene

TABLE I

| Example No. —— | 1 | 2 | 3 | 4 | 5 |
|---|---|---|---|---|---|
| Potassium hydroxide | 19.47g. | 19.47g. | 19.47g. | 19.47g. | 19.47g. |
| % Neutralization | 85% | 85% | 85% | 85% | 85% |
| Water | 10.88g. | 10.88g. | 10.88g. | 10.88g. | 10.88g. |
| Acrylic acid | 25.0g. | 25.0g. | 25.0g. | 25.0g. | 25.0g. |
| Comonomer | A 3.75g. | B 3.75g. | C 3.75g. | D 3.75g. | E 3.75g. |
| AEROSOL A102[1] | 3.46g. | 3.46g. | 3.46g. | 3.46g. | 3.46g. |
| IRGACURE 651[2] | 0.35g. | 0.35g. | 0.35g. | 0.35g. | 0.35g. |

[1]AEROSOL A102 is disodium ethoxylated alcohol half ester of sulfosuccinic acid, a dispersant.

[2]IRGACURE 651 is 2,2-dimethoxy-2-phenylacetophenone, a photoinitiator.

-16-

0047009

TABLE I (Continued)

| Example No. ——— | 6 | 7 |
|---|---|---|
| Sodium hydroxide | 4.36g. | 4.36g. |
| % Neutralization | 95% | 95% |
| Water | 17.21g. | 17.21g. |
| Acrylic acid | 8.3g. | 8.3g. |
| Comonomer | F | A |
| | 1.7g. | 1.7g. |
| AEROSOL A102[1] | 0.12g. | 0.12g. |
| IRGACURE 651[2] | 0.12g. | 0.12g. |

-18-

Copolymers having substantially the same properties are obtained when in some of the above compositions potassium hydroxide is replaced with ammonium hydroxide, IRGACURE 651 is replaced with 2,2-diethyoxyacetophenone or photoinitiator E-7552, a benzoin ether, sold by Stauffer Chemical Co., and AEROSOL A102 is replaced with an oligomeric surfactant POLYWET KX-3 (from Uniroyal) or TRITON N-111 (mono-phenoxy polyethoxy ethanol).

A number of tests are available to determine the absorbency of a material. Following are descriptions of test procedures which were employed in evaluating absorbency of the interpolymers of this invention.

Static Test (ST) - A weighed film sample is immersed in a test liquid for 10 minutes. It is then removed from the liquid, the excess liquid drained for a few seconds and then shaken lightly several times. The swelled sample is weighed again to determine the weight of liquid absorbed by the polymer.

Demand Absorbency Test (DAT) - A test diaper is constructed from a 4 inch diameter pad (10.16 cm.) using materials from a commercial diaper. A film prepared from a polymer to be tested for absorbency is placed in the center of the test diaper between two layers of fluff (wood pulp). A diaper without the polymer film is used as a blank. The demand absorbency apparatus is a burette filled with the test fluid and firmly stoppered at the top, with an air bleed on the side, and a delivery orifice on the bottom connected by a flexible tube to the sample holder. The sample holder has an opening in the center which is connected to the flexible tube that leads to the delivery orifice of the burette. The sample holder is level with the air bleed opening in the burette. With this closed-system arrangement the fluid in the flexible tube that comes up to the opening in the sample holder is at zero pressure. Thus when the test diaper is placed on the sample holder over the opening it will absorb the fluid on its own through wicking action. The sample's own absorbent powder will determine the rate and amount of fluid that will be withdrawn from the burette. The amount of fluid withdrawn at any given time can be easily read from the burette calibration. An additional feature is that absorbency can be measured against a range of pressures that can be obtained by placing various weights on top of the test diaper. Such pressures are intended to simulate the pressures applied on a diaper in actual use.

This test is described in greater detail by Lichstein, "Demand Wettability, a New Method for Measuring Absorbency Characteristics of Fabrics", Symposium Papers-INDA Technical Symposium, 1974, pp. 129-142.

Compression Test (CT) - This test is a follow-up test to the Demand-Absorbency Test (DAT). After the sample has absorbed the liquid against a lower pressure in a DAT, it is removed from the DAT apparatus and placed atop a porous filter funnel. The sample is then subjected to 1.5 psi ($0.105$ kg/cm$^2$) of pressure for 1 minute and the amount of liquid that is squeezed from the sample is measured. Said pressure corresponds to the maximum pressure that is exerted on portions of a diaper when a toddler is picked up or held. This is 10 to 15 times the pressure that the diaper normally would experience. The sample is then weighed to determine the amount of fluid in grams retained per one gram of polymer.

In Table II below is presented data showing absorbency properties of the copolymers of this invention. The polymer number in the Table corresponds to the example describing the preparation of that specific polymer.

BAD ORIGINAL

-20-

TABLE II

| Polymer | ST (g/g) | DAT (ml/g) | CT (g/g) |
|---|---|---|---|
| 1 | -- | 20.2 | -- |
| 2 | -- | 24.5 | -- |
| 3 | -- | 28.8 | -- |
| 4 | -- | 28.0 | -- |
| 5 | -- | 12.5 | -- |
| 6 | 34 | 15 | 14 |
| 7 | 70 | 34 | 31 |

-21-

## CLAIMS

1. An interpolymer of

    a) 70 to 93 weight percent of acrylic acid, 70 to 100 percent of the carboxylic groups having been neutralized with an alkali metal hydroxide or ammonia base prior to polymerization, and

    b) 7 to 30 weight percent of a comonomer selected from the group consisting of an $\alpha$-olefin of 6 to 18 carbon atoms, styrene and a substituted styrene.

2. An interpolymer of Claim 1 wherein 80 to 100 percent of carboxylic groups are neutralized.

3. An interpolymer of Claim 2 obtained from photopolymerization of said monomers in the presence of from 0.01 to 5 weight percent of a photoinitiator.

4. An interpolymer of Claim 3 wherein the comonomers are selected from the group consisting of styrene, vinyl toluene, p-chlorostyrene, p-t-butylstyrene, and $\alpha$-olefin having 8 to 18 carbon atoms, and a mixture thereof.

5. An interpolymer of Claim 1 obtained from radiation polymerization of said monomers by subjecting them to electron beam radiation of sufficient intensity and quantity to cause said monomers to polymerize substantially completely.

6. An interpolymer of Claim 5 wherein the comonomer is selected from the group consisting of styrene, vinyl toluene, p-chlorostyrene, p-t-butyl-styrene, and $\alpha$-olefin having 8 to 18 carbon atoms, and a mixture thereof.

7. An interpolymer of Claim 1 which contains up to 5 weight percent of an additional comonomer selected from an acrylate, a methacrylate or an ethylenically unsaturated nitrile.

0047009

-22-

8. An interpolymer of Claim 1 containing 80 to 95 weight percent of acrylic monomer units and 5 to 20 weight percent of a comonomer.

9. An article of manufacture designed for absorbing body fluids comprising an absorbent cellulose material and a polymer of Claim 1 in the form of a film or fibers.

European Patent Office

**EUROPEAN SEARCH REPORT**

0047009

Application number

EP 81 10 6798

## DOCUMENTS CONSIDERED TO BE RELEVANT

| Category | Citation of document with indication, where appropriate, of relevant passages | Relevant to claim |
|---|---|---|
| | US - A - 3 755 272 (W.J. BLANK) <br> * Claim 1 * | 1,2,8 |
| | -- | |
| | CHEMICAL ABSTRACTS, vol. 70, 1969 page 5, abstract 47931q Columbus, Ohio, US MUSAEV, U.N. et al. "Radiation copolymerization of styrene with methacrylic acid" <br> & IZV. VYSSH. UCHEB. ZAVED. KHIM. KHIM. TEKHNOL. 1968, 11(10), 1170-1173. <br> * Abstract * | 1,2,8 |
| | -- | |
| D | US - A - 4 167 464 (P.J. GEORGE) <br> * Claim 1 * | 1-5, 7,9 |
| | ----- | |

**CLASSIFICATION OF THE APPLICATION (Int Cl.)**

C 08 F 220/06
A 61 L 15/00
C 08 F 2/46/
(C 08 F 220/06
212/00
210/14)

**TECHNICAL FIELDS SEARCHED (Int. Cl.)**

C 08 F 20/04
20/06
220/04
220/06
2/46
2/50
A 61 L 15/00

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 30-11-1981 | CAUWENBERG |

EPO Form 1503.1 06.78